# EUROPÄISCHE PATENTSCHRIFT

(11) **EP 0 640 597 B1**
(45) Veröffentlichungstag und Bekanntmachung des Hinweises auf die Patenterteilung: **23.06.1999**
(21) Anmeldenummer: 94112953.8
(22) Anmeldetag: 19.08.1994
(51) Int. Cl.: C07D 275/03, C07D 261/18, C07D 261/14, A01N 43/80, C07D 413/12, C07D 417/12

(54) **Acylamino-substituierte Isoxazol- bzw. Isothiazolderivate, Verfahren zu deren Herstellung und ihre Verwendung**
Acylamino substituted isoxazoles and/or isothiazols, process for their preparation and their use
Isoxazoles et/ou isothiazoles, substitués par un groupe acylamino, leur preparation et utilisation

(30) Priorität: 24.08.1993 DE 4328425
(43) Veröffentlichungstag der Anmeldung: 01.03.1995
(73) Patentinhaber: BASF Aktiengesellschaft, 67063 Ludwigshafen (DE)
(72) Erfinder: Muenster, Peter, Dr., D-68809 Neulussheim (DE); Schefczik, Ernst, Dr., D-67069 Ludwigshafen (DE); Koenig, Hartmann, Dr., D-67117 Limburgerhof (DE); Gerber, Matthias, Dr., D-67117 Limburgerhof (DE); Westphalen, Karl-Otto, Dr., D-67346 Speyer (DE); Walter, Helmut, Dr., D-67283 Obrigheim (DE)

(56) Entgegenhaltungen:
- EP-A- 0 044 185
- CH-A- 478 133
- DE-A- 1 954 179
- FR-A- 2 237 893
- US-A- 3 770 740
- US-A- 4 059 433
- BERICHTE DER DEUTSCHEN CHEMISCHEN GESELLSCHAFT, Bd.96, Nr.2, 1963, WEINHEIM DE Seiten 526 - 533 JOACHIM GOERDELER UND AL 'Darstellung und Cyclisierung von alpha-Acyl-beta-amino-thiocrotonamide'
- TETRAHEDRON, Bd.23, Nr.2, 1967, OXFORD GB Seiten 687 - 691 G. DESIMONI ET AL 'Polynuclear isoxazole types'
- CHEMICAL ABSTRACTS, vol. 116, no. 19, 11. Mai 1992, Columbus, Ohio, US; abstract no. 194196a, CHIARA B. VICENTINI ET AL 'Fungitoxicity of 5_aminoisoxazole-4-thiocyanate derivatives' Seite 716 ; & PESTIC.SCI., Bd.34, Nr.2, 1992 Seiten 127 - 131
- CHEMICAL ABSTRACTS, vol. 80, no. 23, 10. Juni 1974, Columbus, Ohio, US; abstract no. 128066v, WOLNA ELZBIETA ET AL 'New isothiazole derivatives' Seite 12 ; & ARCH. IMMUNOL. THER. EXP., Bd.21, Nr.6, 1973 Seiten 909 - 914
- CHEMICAL ABSTRACTS, vol. 74, no. 17, 26. April 1971, Columbus, Ohio, US; abstract no. 87882p, ZDZISLAW MACHON ET AL 'Synthesis and biological properties of derivatives of 3-methyl-5-aminoisothiazole-4-carboxylic acid' Seite 430 ; & DISS PHARM. PHARMACOL., Bd.22, Nr.6, 1970 Seiten 395 - 402

## Beschreibung

Die vorliegende Erfindung betrifft neue Acylamino-substituierte Isoxazol- bzw. Isothiazol-Derivate der allgemeinen Formel I 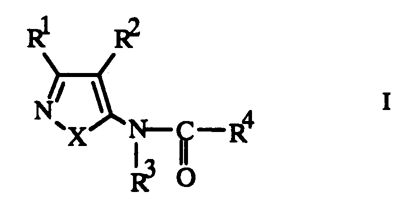 in der die Variablen folgende Bedeutung haben:
X
   Sauerstoff oder Schwefel;
R¹
   C₁-C₆-Alkyl, welches ein bis fünf Halogenatome und/oder einen Cyanorest und/oder bis zu zwei der folgenden Reste tragen kann: C₁-C₄-Alkoxy, C₁-C₄-Halogenalkoxy, C₁-C₄-Alkylthio, C₁-C₄-Halogenalkylthio,
   C₃-C₆-Cycloalkyl, welches wiederum durch ein bis drei Halogen- oder C₁-C₄-Alkylreste substituiert sein kann oder Phenyl, welches noch einen bis drei der folgenden Reste tragen kann: Cyano, Halogen, Nitro, C₁-C₆-Alkyl, C₁-C₆-Halogenalkyl, C₁-C₆-Alkoxy, C₁-C₆-Halogenalkyl, C₁-C₆-Alkylthio, C₁-C₆-Halogenalkylthio;
   C₂-C₆-Alkenyl, dessen Doppelbindung epoxidiert sein kann, oder eine C₂-C₆-Alkinylgruppe, wobei beide Gruppen ein- bis dreimal durch Halogen, C₁-C₃-Alkoxy und/oder einmal durch Cyclopropyl oder Phenyl substituiert sein können, wobei der Phenylrest zusätzlich ein bis drei der folgenden Substituenten tragen kann: Halogen, Cyano, Nitro, C₁-C₄-Alkyl, C₁-C₄-Halogenalkyl, C₁-C₄-Alkoxy,
   C₁-C₄-Halogenalkoxy, C₁-C₄-Alkylthio, C₁-C₄-Halogenalkylthio; C₃-C₈-Cycloalkyl oder C₃-C₆-Cycloalkenyl, wobei beide Gruppen einbis dreimal durch C₁-C₄-Alkyl und/oder Halogen substituiert sein können;
R²
   CN, CONH₂, CSNH₂;
R³
   Wasserstoff;
   C₁-C₆-Alkyl, das einen bis drei der folgenden Substituenten tragen kann: Hydroxy, Halogen, C₁-C₄-Alkoxy und/oder C₁-C₄-Alkylthio;
   C₃-C₈-Cycloalkyl, das ein- bis dreimal durch Halogen, C₁-C₄-Alkyl und/oder C₁-C₄-Halogenalkyl substituiert sein kann;
R⁴
   eine C₁-C₄-Alkoxygruppe;
   eine C₁-C₆-Alkylgruppe, die einen bis drei der folgenden Reste tragen kann: Halogen, Cyano, C₁-C₄-Alkoxy, C₁-C₄-Halogenalkoxy, C₁-C₄-Alkylthio, C₁-C₄-Halogenalkylthio, C₃-C₈-Cycloalkyl oder Phenyl, wobei der Phenylring seinerseits einen bis drei der folgenden Reste tragen kann: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio;
   eine C₃-C₆-Cycloalkenylgruppe, die ein- bis dreimal durch Halogen oder C₁-C₄-Alkyl substituiert sein kann;
   eine C₃-C₈-Cycloalkylgruppe, die einen bis drei der folgenden Reste tragen kann: Halogen, Nitro, Cyano, C₁-C₆-Alkyl, C₁-C₆-Halogenalkyl, C₁-C₄-Alkoxy und/oder C₁-C₄-Halogenalkoxy;
   eine C₃-C₆-Alkenyl- oder C₃-C₆-Alkinylgruppe, die jeweils ein- bis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
   die Phenylgruppe, welche noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio;
   und landwirtschaftlich brauchbare Salze der Isoxazol- bzw. Isothiazolderivate I, ausgenommen:
   3-Methyl-4-cyano-5-benzoylamino-isothiazol,
   3-Methyl-4-carboxamido-5-acetylamino-isothiazol,
   3-Methyl-4-carboxamido-5-(4-chlorbenzoyl)-amino-isothiazol,
   3-Methyl-4-carboxamido-5-(2-chlorbenzoyl)-amino-isothiazol,
   3-Methyl-4-carboxamido-5-benzoylamino-isothiazol.

Es ist allgemein bekannt, daß Harnstoffverbindungen gute herbizide Eigenschaften aufweisen. Nach K.H. Büchel "Pflanzenschutz und Schädlingsbekämpfung", Georg Thieme Verlag, Stuttgart 1977, Seiten 169-172 ist die herbizide Aktivität dieser Verbindungsklasse besonders gut, wenn trisubstituierte Harnstoffe vorliegen. Harnstoffderivate mit heterocyclischen Substituenten sind z.B. in DE-A 24 34 922 beschrieben.

Ferner ist bekannt, daß bestimmte substituierte Isoxazol- und Isothiazolderivate herbizide Wirkungen zeigen. Beispiele für solche Verbindungen sind 3-Chlor-4-cyano-5-pivaloylamino-isothiazol (DE-A 19 24 830) und 3-Isopropoxy-4-cyano-5-isobuturylamino-isothiazol (US-A 4,059,433).

In EP-A-0 044 185 werden Isoxazolderivate beschrieben, die durch einen Imidazolinonrest substituiert sind. Diese Verbindungen zeigen eine herbizide Wirkung gegen Boden- und Wasserunkräutern.

In DE-A-19 54 179 und CH-A-478 133 werden Isothiazolderivate beschrieben, in denen R¹ Chlor bedeutet. Diese Verbindungen werden als Wirkstoffe zur Herstellung von Schädlingsbekämpfungsmittel verwendet.

Die Wirkung bzw. Kulturpflanzenverträglichkeit dieser Verbindungen ist jedoch nicht in jedem Fall ausreichend.

Isoxazole mit fungiziden Eigenschaften werden in JP-A 59/128 306 (z.B. 3-Phenyl-4-chlor-5-chloracetylamino-isoxazol) und EP-A 105 548 (z.B. 3-Phenyl-4-chlor-5-acetylaminoisoxazol) offenbart.

Ferner sind in der Literatur folgende Verbindungen ohne Angabe von herbiziden Eigenschaften beschrieben:
3-Methyl-4-rhodano-5-trifluoracetylamino-isoxazol und 3-Methyl-4-rhodano-5-benzoylamino-isoxazol (Pestic. Sci. 34, 127 (1992)), 3-Methyl-4-cyano-5-(2-furyl)-carbonylamino-isoxazol und 3-Ethyl-4-cyano-5-(2-furyl)-carbonylamino-isoxazol (US-A 3,770,740 bzw. J. Med. Chem. 17, 451 (1974)), 3-Methyl-4-cyano-5-benzoylamino-isothiazol (Chem. Ber. 96, 526 (1963)), 3-Methyl-4-carboxamido-5-acetylamino-isothiazol und 3-Methyl-4-carboxamido-5-benzoylamino-isothiazol (Arch. Immunol. Ther. Exp. 21, 909 (1973), CA 80: 128066), 3-Methyl-4-carboxamido-5-(4-chlorbenzoyl)-amino-isothiazol und 3-Methyl-4-carboxamido-5-(2-chlorbenzoyl)-amino-isothiazol (Diss. Pharm. Pharmacol. 22, 395 (1970), CA 74: 87882), 3-Phenyl-4-cyano-5-benzoylamino-isoxazol, 3-Phenyl-4-cyano-5-(4-chlorbenzoyl)-amino-isoxazol, 3-Phenyl-4-cyano-5-(4-methylbenzoyl)-amino-isoxazol und 3-Phenyl-4-cyano-5-propionylamino-isoxazol (Tetrahedron 23, 687 (1967)).

Es bestand nun die Aufgabe, Isoxazol- bzw. Isothiazolderivate mit verbesserten Eigenschaften insbesondere im Hinblick auf Wirksamkeit und Kulturpflanzenverträglichkeit zu synthetisieren. Demgemäß wurden die eingangs definierten Verbindungen der allgemeinen Formel I gefunden.

Die erfindungsgemaßen Verbindungen der Formel I können auf verschiedenen Wegen hergestellt werden.

Es wurde gefunden, daß die neuen Isoxazol- bzw. Isothiazolderivate der Formel I, 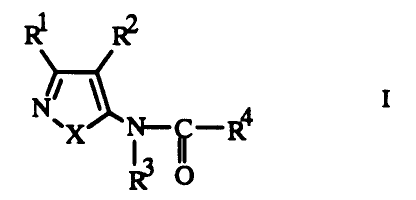 in der die Reste R³ Alkyl bzw. Cycloalkyl und X, R¹, R² und R⁴ die eingangs genannten Bedeutungen haben, dadurch erhalten werden, daß man eine Verbindung der Formel II, 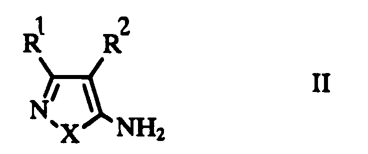 in der X, R¹ und R² die voranstehend genannten Bedeutungen haben, zunächst mit einem Carbonsäurechlorid der Formel III, 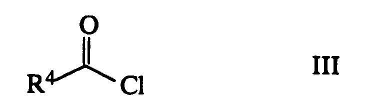 in der R⁴ die oben genannte Bedeutung hat, oder einem anderen aktivierten Derivat einer Carbonsäure, wie z.B. dem Carbonsäureanhydrid, zu Verbindungen der Formel IV umsetzt (Reaktionsschritt A), 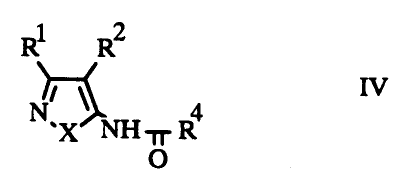 und diese mit einer Alkyl- bzw. Cycloalkylhalogenverbindung der Formel V

R³-Hal V

in der R³ für ggf. substituiertes Alkyl oder Cycloalkyl wie voranstehend genannt und Hal für Halogen, z.B. Chlor oder Brom, steht, bzw. einem Sulfat der allgemeinen Formel VI

(R³O)₂SO₂ VI

gegebenenfalls in Gegenwart einer Base umsetzt (Reaktionsschritt B).

Die einzelnen Reaktionsschritte können wie folgt durchgeführt werden:

### Reaktionsschritt A:

Zweckmäßigerweise geht man so vor, daß man die Reaktionspartner in einem inerten organischen Lösungsmittel bzw. Lösungsmittelgemisch bei Temperaturen von -20°C bis zum Siedepunkt des Lösungsmittels gegebenenfalls in Gegenwart einer Base zur Reaktion bringt. Das Verhältnis von Amin II zu aktivierter Carbonsäure III kann zwischen 2,5 : 1 und 1 : 3 gewählt werden. Wird z.B. ein Carbonsäurechlorid äquimolar oder im Überschuß eingesetzt, ist es sinnvoll, die Reaktion in Gegenwart einer Hilfsbase durchzuführen. Hierzu eignen sich z.B. Amine wie Triethylamin, Pyridin, oder auch Alkali- bzw. Erdalkalihydroxide. Auch der Zusatz einer katalytischen Menge N,N-Dimethylaminopyridin kann vorteilhaft sein. Geeignete Lösungsmittel für die Reaktionen sind unter anderem Kohlenwasserstoffe wie Benzol, Toluol, Xylol, Halogenkohlenwasserstoffe wie Dichlorethan oder auch Ether wie Diethylether, tert.-Butylmethylether oder Tetrahydrofuran.

### Reaktionsschritt B:

Die Alkylierung des Amids erfolgt zweckmäßigerweise so, daß man die Verbindung der allgemeinen Formel IV in einem aprotischen organischen Lösungsmittel bei Temperaturen zwischen -10°C und +50°C in Gegenwart einer Base mit einem Alkylierungsmittel der allgemeinen Formel V oder VI umsetzt. Dabei kann das Alkylierungsmittel äquimolar oder in einem Überschuß, z.B. bis zu 2-fachen Überschuß eingesetzt werden. Geeignete Lösungsmittel sind Kohlenwasserstoffe wie Benzol, Toluol oder Xylol, Ether wie Diethylether, tert.-Butylmethylether, Tetrahydrofuran, halogenierte Kohlenwasserstoffe wie Dichlormethan, DMF oder auch DMSO. Als Basen eignen sich Alkali- und Erdalkalicarbonate, -hydoxide oder -hydride wie Natrium- bzw. Kaliumcarbonat oder -hydroxid oder Natriumhydrid, Alkoholate wie Natriummethanolat oder Metallorganyle wie n-Butyllithium.

Geeignete Alkylierungsmittel sind Alkyl- bzw. Cycloalkylhalogenide wie z.B. Methyliodid oder auch Dimethylsulfat.

Eine Möglichkeit, Verbindungen der allgemeinen Formel I, in der R² CONH₂ und X, R¹, R³ und R⁴ die oben angegebenen Bedeutungen haben, zu synthetisieren, besteht darin, ein Nitril der allgemeinen Formel VII 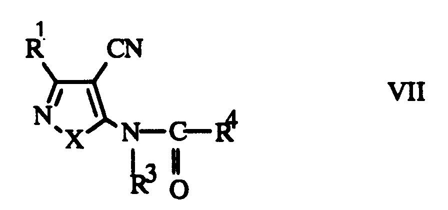 zu hydrolysieren. Diese Hydrolyse kann z.B. in saurem Medium durchgeführt werden. Geeignet sind starke Säuren wie konzentrierte Schwefelsäure, Polyphosphorsäuren, Chlorwasserstoff in Ameisensäure oder Eisessig oder Bortrifluorid/Eisessig.

Desweiteren ist es möglich, die Nitrile in alkalischem Medium zu den Amiden zu hydrolysieren. Geeignete Reagenzien sind H₂O₂ mit Natronlauge oder Natriumcarbonat. Diese Reaktion kann auch vorteilhaft unter Bedingungen der Phasentransferkatalyse durchgeführt führt werden. Kaliumhydroxid/tert.-Butanol, Ammoniak, Natriumperoxid/DMSO oder ein stark alkalischer Ionenaustauscher können ebenfalls eingesetzt werden. Alle Hydrolysen verlaufen vorteilhaft im Temperaturbereich von -20°C bis 100°C. Die Reagenzien werden äquimolar oder im Überschuß zugegeben. Sehr häufig verlaufen die Hydrolysen in Abwesenheit organischer Lösungsmittel. Unter den Bedingungen der Phasentransferkatalyse sind halogenierte Kohlenwasserstoffe wie Dichlormethan geeignete Lösungsmittel.

Verbindungen der allgemeinen Formel I, in denen R² CSNH₂ bedeutet und X, R¹, R³ und R⁴ die oben angegebene Bedeutung haben, erhält man, indem man ein Nitril der allgemeinen Formel VII, in einem organischen Lösungsmittel bei Temperaturen zwischen 0 und 100°C und Normal- bzw. erhöhtem Druck, ggf. in Gegenwart einer katalytischen oder äquimolaren Menge einer Stickstoffbase mit Schwefelwasserstoff behandelt. Geeignete Lösungsmittel für diese Umsetzung sind Alkohole wie Methanol, Ethanol oder Isopropanol, Ether, aromatische Kohlenwasserstoffe wie Benzol, Toluol oder Xylol, Pyridin oder andere polare Lösungsmittel wie z.B. N-Methylpyrrolidon. Als Basen können tertiäre Amine wie z.B. Triethylamin oder Pyridin eingesetzt werden.

Im Hinblick auf die erfindungsgemäße herbizide Wirkung haben die Substituenten in den Verbindungen I beispielsweise die folgende Bedeutung:
X
   Sauerstoff oder Schwefel;
R¹
   unverzweigtes oder verzeigtes C₁-C₆-Alkyl, wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl, Ethyl, Propyl, 1-Methylethyl und 1,1-Dimethylethyl, welches ein bis fünf Halogenatome wie Fluor, Chlor, Brom, Jod, insbesondere Fluor und Chlor, und/oder einen Cyanorest und/oder bis zu zwei der folgenden Reste tragen kann:
   C₁-C₄-Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;
   partiell oder vollständig halogeniertes C₁-C₄-Alkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;
   C₁-C₄-Alkylthio wie Methylthio, Ethylthio, Propylthio, 1-Methylethylthio, n-Butylthio, 1-Methylpropylthio, 2-Methylpropylthio und 1,1-Dimethylethylthio, insbesondere Methylthio und Ethylthio;
   partiell oder vollständig halogeniertes C₁-C₄-Alkylthio wie Difluormethylthio, Trifluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2-fluorethylthio, 2,2,2-Trichlorethylthio und Pentafluorethylthio, insbesondere Trifluormethylthio und Pentafluorethylthio;
   eine C₃-C₈-Cycloalkylgruppe wie Cyclopropyl, Cyclobutyl, Cyclopentyl, Cyclohexyl, Cycloheptyl und Cyclooctyl, insbesondere Cyclopropyl, Cyclopentyl und Cyclohexyl, wobei der Cyclus noch ein- bis dreimal durch C₁-C₄-Alkyl wie vorstehend genannt, insbesondere Methyl und Ethyl; oder Halogen wie Fluor, Chlor, Brom und Jod, insbesondere Fluor und Chlor, substituiert sein kann;
   R¹ ferner eine C₂-C₆-Alkenylgruppe, deren Doppelbindung epoxidiert sein kann, bevorzugt eine C₂-C₄-Alkenylgruppe wie Ethenyl, Prop-2-en-1-yl, 1-Methylethenyl, But-2-en-1-yl und 1-Methylprop-2-en-1-yl, welche ein- bis dreimal durch Halogen wie Fluor, Chlor, Brom oder Jod, C₁-C₃-Alkoxy wie Methoxy, Ethoxy oder Isopropoxy und/oder einmal durch Cyclopropyl oder Phenyl substituiert sein kann, wobei der Phenylrest seinerseits eine bis drei der folgenden Gruppen tragen kann: Cyano, Nitro, Alkyl wie vorstehend genannt, insbesondere Methyl, Ethyl und 1-Methylethyl; Halogenalkyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; partiell oder vollständig halogeniertes Alkylthio wie vorstehend genannt, insbesondere Difluormethylthio, Trifluormethylthio und Pentafluormethylthio oder Halogen wie vorstehend genannt, insbesondere Fluor und Chlor;
   R¹ ferner eine C₂-C₆-Alkinylgruppe, bevorzugt eine C₂-C₄-Alkinylgruppe wie Ethinyl, Propin-1-yl, 1-Methyl-2-propinyl und n-Butinyl, welche ein- bis dreimal durch Halogen wie Fluor, Chlor, Brom oder Jod, C₁-C₃-Alkoxy wie Methoxy oder Isopropoxy und/oder einmal durch Cyclopropyl oder Phenyl substituiert sein kann, wobei der Phenylrest seinerseits eine bis drei der folgenden Gruppen tragen kann: Cyano, Nitro, Alkyl wie vorstehend genannt, insbesondere Methyl, Ethyl und 1-Methylethyl; Halogenethyl wie vorstehend genannt, insbesondere Trifluormethyl und Chlordifluormethyl; Alkoxy wie vorstehend genannt, insbesondere Methoxy und Ethoxy; Halogenalkoxy wie vorstehend genannt, insbesondere Trifluormethoxy, Trichlormethoxy und Pentafluorethoxy; Alkylthio wie vorstehend genannt, insbesondere Methylthio und Ethylthio; partiell oder vollständig halogeniertes Alkylthio wie vorstehend genannt, insbesondere Difluormethylthio, Trifluormethylthio und Pentafluormethylthio oder Halogen wie vorstehend genannt, insbesondere Fluor und Chlor;
   R¹ ferner eine C₃-C₈-Cycloalkylgruppe wie Cyclopropyl, Cyclobutyl, Cyclopentyl, Cyclohexyl, Cycloheptyl und Cyclooctyl, insbesondere Cyclopropyl, Cyclopentyl und Cyclohexyl oder eine C₃-C₆-Cycloalkenylgruppe, insbesondere eine C₅-C₆-Cycloalkenylgruppe wie Cyclohexen-1-yl, wobei der Cyclus noch ein- bis dreimal durch C₁-C₄-Alkyl wie vorstehend genannt, insbesondere Methyl und Ethyl;
   oder Halogen wie Fluor, Chlor, Brom und Jod, insbesondere Fluor und Chlor, substituiert sein kann;
R²
   CN, CONH₂, CSNH₂;
R³
   Wasserstoff;
   C₁-C₆-Alkyl, bevorzugt C₁-C₄-Alkyl wie Methyl, Ethyl und tert.-Butyl, das einen bis drei der folgenden Substituenten tragen kann: Hydroxy, Halogen, C₁-C₄-Alkoxy wie Methoxy und tert.-Butoxy, C₁-C₄-Alkylthio wie Methylthio und tert.-Butylthio;
   C₃-C₈-Cycloalkyl, bevorzugt C₅-C₆-Cycloalkyl wie Cyclopentyl und Cyclohexyl, das ein- bis dreimal durch Halogen wie Fluor, Chlor und Brom, C₁-C₄-Alkyl wie Methyl und tert.-Butyl oder partiell oder vollständig halogeniertes C₁-C₄-Alkyl wie Fluormethyl, Trifluormethyl, Chlordifluormethyl, Pentafluorethyl und 2-Chlor-1,1,2-trifluorethyl substituiert sein kann;
R⁴
   C₁-C₄-Alkoxy, wie vorstehend genannt, insbesondere Methoxy, Ethoxy und Isopropoxy;
   verzweigtes oder unverzweigtes C₁-C₆-Alkyl, bevorzugt C₂-C₄-Alkyl wie Ethyl, Isopropyl und tert.-Butyl, welches einen bis drei der folgenden Reste tragen kann: Halogen wie Fluor, Chlor und Brom, Cyano, C₁-C₄-Alkoxy wie Methoxy und tert.-Butoxy, partiell oder vollständig halogeniertes C₁-C₄-Alkoxy wie Fluormethoxy, Trichlormethoxy, 2-Chlor-1,1,2-trifluorethoxy und Pentafluorethoxy,
   C₁-C₄-Alkylthio wie Methylthio und tert.-Butylthio, C₁-C₄-Halogenalkylthio wie Fluormethylthio, Trichlormethylthio, 2-Chlor-1,1,2-trifluorethylthio und Pentafluorethylthio, C₃-C₈-Cycloalkyl, insbesondere C₃-C₆-Cycloalkyl wie Cyclopropyl, Cyclopentyl und Cyclohexyl oder Phenyl, wobei der Phenylrest seinerseits bis zu drei der folgenden Gruppen tragen kann: Halogen wie Fluor, Chlor und Brom, Cyano, Nitro, C₁-C₄-Alkyl wie Methyl und tert.-Butyl, C₁-C₄-Halogenalkyl wie Fluormethyl, Trichlormethyl, 2-Chlor-1,1,2-trifluorethyl und Pentafluorethyl, C₁-C₄-Alkoxy wie Methoxy und tert.-Butoxy, partiell oder vollständig halogeniertes C₁-C₄-Alkoxy wie Fluormethoxy, Trichlormethoxy, 2-Chlor-1,1,2-trifluorethoxy und Pentafluorethoxy, C₁-C₄-Alkylthio wie Methylthio und tert.-Butylthio oder C₁-C₄-Halogenalkylthio wie Fluormethylthio, Trichlormethylthio, 2-Chlor-1,1,2-trifluorethylthio und Pentafluorethylthio;
   R⁴ ferner C₃-C₈-Cycloalkyl, bevorzugt C₃-C₆-Cycloalkyl, insbesondere Cyclopropyl, Cyclopentyl und Cyclohexyl, das jeweils einen bis drei der folgenden Reste tragen kann: Halogen wie Fluor, Chlor und Brom, Nitro, Cyano, C₁-C₆-Alkyl, bevorzugt C₁-C₄-Alkyl wie Methyl und tert.-Butyl, partiell oder vollständig halogeniertes C₁-C₆-Alkyl, bevorzugt C₁-C₄-Halogenalkyl wie Flourmethyl, Trichlormethyl, 2-Chlor-1,1,2-trifluorethyl und Pentafluorethyl, C₁-C₄-Alkoxy wie Methoxy und tert.-Butoxy, oder partiell oder vollständig halogeniertes C₁-C₄-Alkoxy wie Fluormethoxy, Trichlormethoxy, 2-Chlor-1,1,2-trifluorethoxy und Pentafluorethoxy;
   eine C₃-C₆-Cycloalkenylgruppe, bevorzugt eine C₅-C₆-Cycloalkenylgruppe, die ein- bis dreimal durch Halogen wie Fluor, Chlor, Brom und Jod, insbesondere Fluor und Chlor, oder C₁-C₄-Alkyl wie Methyl, Ethyl und tert.-Butyl substituiert sein kann;
   R⁴ ferner C₃-C₆-Alkenyl oder C₃-C₆-Alkinyl, bevorzugt C₃-C₄-Alkenyl oder C₃-C₅-Alkinyl wie 2-Propenyl, 2-Butenyl, 2-Propinyl, 1,1-Dimethyl-2-propinyl und 3-Butinyl, welches jeweils ein- bis dreifach durch Halogen wie Fluor, Chlor oder Brom und/oder einmal durch Phenyl substituiert sein kann, wobei der Phenylrest seinerseits eine bis drei der folgenden Substituenten tragen kann: Halogen, insbesondere Fluor und Chlor, Cyano, Nitro, C₁-C₄-Alkyl wie Methyl und tert.-Butyl, partiell oder vollständig halogeniertes C₁-C₄-Alkyl wie Fluormethyl, Trifluormethyl, Trichlormethyl, 2-Chlor-1,1,2-trifluorethyl und Pentafluorethyl, C₁-C₄-Alkoxy wie Methoxy und tert.-Butoxy, C₁-C₄-Halogenalkoxy wie Fluormethoxy, Trifluormethoxy, Trichlormethoxy, 2-Chlor-1,1,2-trifluorethoxy und Pentafluorethoxy, C₁-C₄-Alkylthio wie Methylthio und tert.-Butylthio, partiell oder vollständig halogeniertes C₁-C₄-Alkylthio wie Fluormethylthio, Trifluormethylthio, Trichlormethylthio, 2-Chlor-1,1,2-trifluorethylthio und Pentafluorethylthio;
   R⁴ ferner die Phenylgruppe, welche noch einen bis drei der folgenden Reste tragen kann: Cyano; Nitro; Halogen wie Fluor, Chlor, Brom und Jod, insbesondere Fluor und Chlor; C₁-C₆-Alkyl wie vorstehend genannt, insbesondere Methyl, Ethyl und 1-Methylethyl; partiell oder vollständig halogeniertes C₁-C₆-Alkyl wie vorstehend genannt, insbesondere Trifluormethyl und Chlordifluormethyl; C₁-C₆-Alkoxy wie vorstehend genannt, insbesondere Methoxy und Ethoxy; partiell oder vollständig halogeniertes C₁-C₆-Alkoxy wie vorstehend genannt, insbesondere Trifluormethoxy, Trichlormethoxy und Pentafluorethoxy; C₁-C₄-Alkylthio wie vorstehend genannt, insbesondere Methylthio und Ethylthio und/oder partiell oder vollständig halogeniertes C₁-C₆-Alkylthio wie vorstehend genannt, insbesondere Difluormethylthio, Trifluormethylthio und Pentafluormethylthio.

Besonders bevorzugt sind Isoxazol- und Isothiazolderivate der Formel I, in der R¹ für eine Alkyl-, Alkenyl- oder Alkinylgruppe mit jeweils 2 bis 6 Kohlenstoffatomen, eine C₃-C₈-Cycloalkyl- oder C₃-C₆-Cycloalkenylgruppe steht, wobei diese Gruppen jeweils wie oben genannt substituiert sein können. Ferner steht R¹ auch vorteilhaft für eine substituierte Methylgruppe, wobei als Substituenten ein bis drei Halogenatome wie Fluor, Chlor oder Brom und/oder eine Cycanogruppe und/oder ein bis zwei der folgenden Reste in Betracht kommen:
C₁-C₄-Alkoxy, C₁-C₄-Halogenalkoxy, C₁-C₄-Alkylthio, C₁-C₄-Halogenalkylthio, C₃-C₆-Cycloalkyl, welches wiederum durch ein bis drei Halogen- und/oder Alkylreste substituiert sein kann oder Phenyl, welches noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio.

Bevorzugte Reste R² sind Cyano, CONH₂ und CSNH₂, bevorzugte Reste R³ Wasserstoff oder C₁-C₆- insbesondere C₁-C₄-Alkyl.

R⁴ steht vorzugsweise für C₁-C₄-Alkoxy, ggf. substituiertes C₁-C₆-Alkyl oder C₃-C₈-Cycloalkyl.

Teil der Erfindung sind auch die landwirtschaftlich brauchbaren Salze von I. Üblicherweise kommt es hierbei auf die Art des Salzes nicht an. Allgemein kommen die Salze von solchen Basen und solchen Estern in Betracht, welche die herbizide Wirkung der Verbindungen I nicht negativ beeinträchtigen.

Landwirtschaftlich brauchbare Salze der Verbindungen I sind beispielsweise Alkalimetallsalze, insbesondere Natrium- und Kaliumsalze, Erdalkalimetallsalze wie insbesondere Calzium-, Magnesium- und Bariumsalze, Mangan-, Kupfer-, Zink- oder Eisensalze sowie Ammoniumsalze wie Tetraalkyl- und Benzyltrialkylammoniumsalze, Phosphonium-, Sulfoniumsalze wie Trialkylsulfoniumsalze oder Sulfoxoniumsalze.

Die Salze der Verbindungen I sind in an sich bekannter Weise zugänglich (EP-A-304 282, US-A 4,599,412). Man erhält sie durch Deprotonierung der entsprechenden Verbindungen I in Wasser oder einem inerten organischen Lösungsmittel bei Temperaturen von -80°C bis 120°C, vorzugsweise 0°C bis 60°C in Gegenwart einer Base.

Geeignete Basen sind beispielsweise Alkali- oder Erdalkalimetallhydroxide, -hydride, -oxide oder -alkoholate wie Natrium-, Kalium- und Lithiumhydroxid, Natriummethanolat, -ethanolat und -tert.-butanolat, Natrium- und Calziumhydrid und Calziumoxid.

Als Lösungsmittel kommen beispielsweise neben Wasser auch Alkohole wie Methanol, Ethanol und tert.-Butanol, Ether wie Tetrahydrofuran und Dioxan, Acetonitril, Dimethylformamid, Ketone wie Aceton und Methylethylketon und auch halogenierte Kohlenwasserstoffe in Betracht.

Die Deprotonierung kann bei Normaldruck oder bei Drucken bis 50 bar, vorzugsweise bei Normaldruck bis 5 bar Überdruck durchgeführt werden.

Die Verbindungen I bzw. die sie enthaltenden herbiziden Mittel sowie deren umweltverträgliche Salze, z.B. von Alkalimetallen und Erdalkalimetallen, können in Kulturen wie Weizen, Reis und Mais, Soja und Baumwolle Schadpflanzen sehr gut bekämpfen, ohne die Kulturpflanzen zu schädigen, ein Effekt, der vor allem auch bei niedrigen Aufwandmengen auftritt. Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohoi, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe wurden dabei in einer Reinheit von 90 % bis 100 %, vorzugsweise 95 % bis 100 % (nach NMR-Spektrum) eingesetzt.

### Beispiele für Formulierungen sind:

I. 20 Gewichtsteile der Verbindung Nr. 1.002 werden in einer Mischung gelost, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
II. 20 Gewichtsteile des Wirkstoffs Nr. 1.003 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 3 Gewichtsteile des Wirkstoffs Nr. 1.002 werden mit 97 Gewichtsteilen feinteiligem Kaolin vermischt. Man erhält auf diese Weise ein Stäubemittel, das 3 Gew.% des Wirkstoffs enthält.
V. 20 Gewichtsteile des Wirkstoffs Nr. 1.003 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 2 kg/ha, vorzugsweise 0,01 bis 1 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

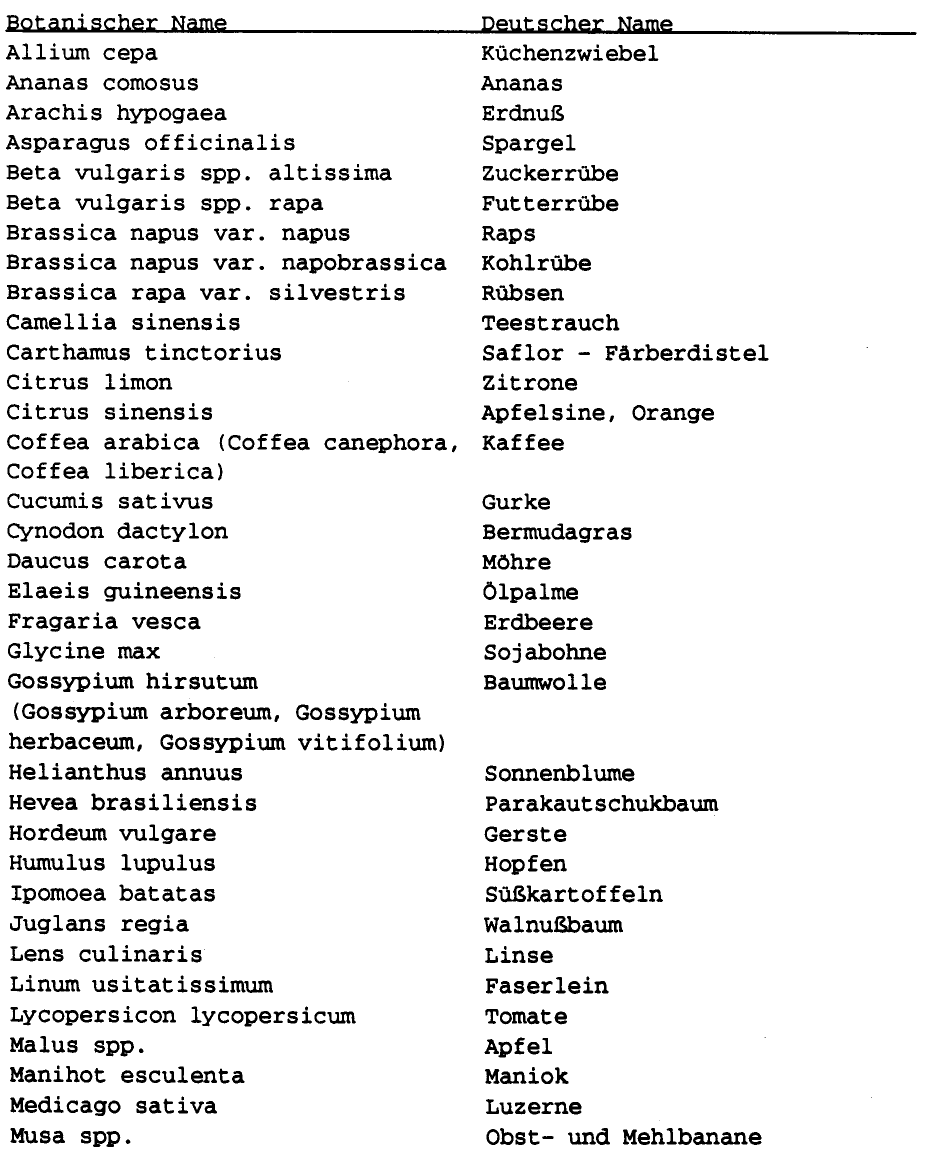

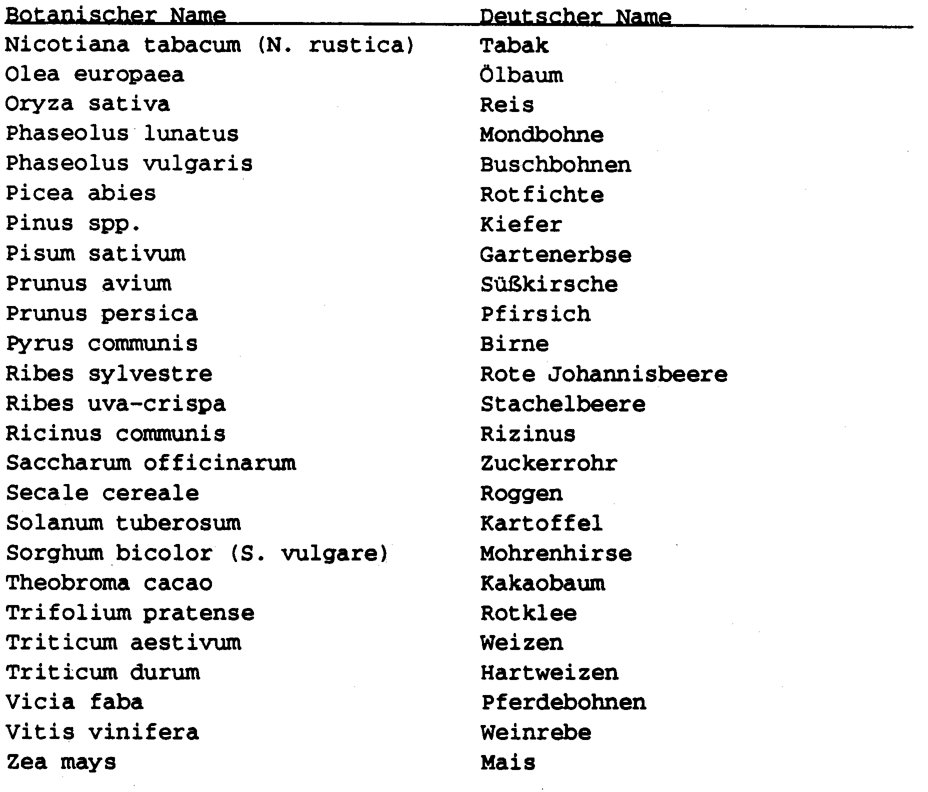

Zur Verbreiterung des Wirkungsspektrums und zur Erzielung synergistischer Effekte können die Acylamino-substituierten Isoxazol- bzw. Isothiazolderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Imidazolinone, Sulfonamide, Sulfonylharnstoffe, Chinolincarbonsäurederivate, Aryloxy- bzw. Heteroaryloxy-phenoxypropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Nachstehend sind typische Beispiele zur Herstellung der Vorprodukte V wiedergegeben.

### Herstellungsbeispiele

### 5-Cyclopropylcarbonylamino-3-isopropyl-isothiazol-4-carbonsäure-nitril

Zu einer Lösung aus 16,7 g 5-Amino-4-cyano-3-isopropylisothiazol und ca. 0,5 g DMAP (N,N-Dimethylaminopyridin) in 200 ml Toluol und 200 ml Pyridin werden 12,5 g Cyclopropancarbonsäurechlorid in 30 ml Toluol getropft. Man läßt die Lösung 16 Stunden bei Raumtemperatur rühren, engt anschließend zur Trockene ein und nimmt den Rückstand in Essigsäureethylester auf. Die organische Phase wird mit wäßriger Natriumhydrogencarbonat-Lösung und 10 %iger Salzsäure extrahiert, getrocknet und eingeengt.

Ausbeute: 22,3 g, Fp. 193°C

### 3-Ethyl-5-(4-chlorbutanoyl)amino-isothiazol-4-carboxamid

5 g 3-Ethyl-5-(4-chlorbutanoyl)amino-isothiazol-4-carbonsäurenitril werden in 30 ml konz. Schwefelsäure 12 Stunden bei Raumtemperatur gerührt. Dann rührt man die Lösung in 250 ml Eiswasser ein, stellt mit konz. Ammoniaklösung schwach alkalisch und extrahiert mit Dichlormethan. Die organische Phase wird getrocknet und eingeengt.

Ausbeute: 3,3 g, Fp. 97°C

### 5-(2-Methylbutanoyl)amino-3-isopropyl-isothiazol-4-thiocarboxamid

8,5 g 5-(2-Methylbutanoyl)amino-3-isopropyl-isothiazol-4-carbonsäurenitril und 3,4 g Triethylamin werden in ca. 60 ml N-Methylpyrrolidon vorgelegt und bei 60°C mit H₂S mehrere Stunden begast. Anschließend läßt man abkühlen, gießt die Lösung auf 1,5 l Wasser und filtriert das Produkt ab.

Ausbeute: 8,8 g, Fp. 132 - 133°C

### 5-(3-Methylbutanoyl)amino-3-isopropyl-isoxazol-4-carboxamid

1,7 g 5-Amino-3-isopropyl-isoxazol-4-carboxamid werden in einem Gemisch aus 40 ml Toluol und 40 ml Pyridin mit einer Spatelspitze Dimethylaminopyridin vorgelegt und mit einer Lösung von 2,5 g Isovaleriansäurechlorid in 20 ml Toluol versetzt. Man läßt 16 Stunden bei Raumtemperatur nachrühren, engt dann zur Trockene ein und verrührt den Rückstand mit Essigsäureethylester. Die organische Phase wird dann mit wäßriger Salzsäure und Natriumhydrogencarbonatlösung extrahiert, getrocknet und eingeengt. Die Reinigung erfolgt über Chromatographie an Kieselgel (Cyclohexan/Essigsäureethylester).

Ausbeute: 0,2 g

¹H-NMR (DMSO-d₆), δ in ppm: 0,95(d,6H); 1,25 (d,6H); 2,05(m,1H); 2,25(d,2H); 3,30(m,1H); 7,40(br,2H); 10,8(s,1H).

### 3-Methyl-5-pivaloylamino-isothiazol-4-isothiocyanat

16,7 g 5-Amino-3-methyl-isothiazol-4-isothiocyanat und 0,5 g Dimethylaminopyridin werden in einem Gemisch aus 100 ml Toluol und 100 ml Pyridin gelöst und mit 15,9 g Pivaloylchlorid versetzt. Man läßt 16 Stunden bei 80°C rühren, engt zur Trockene ein, nimmt in Essigsäureethylester auf und extrahiert mit wäßriger Natriumhydrogencarbonatlösung und 10 %iger Salzsäure. Die organische Phase wird getrocknet und eingeengt.

Ausbeute: 28 g, Fp. 98 - 100°C

In analoger Weise wurden die in nachstehender Tabelle aufgeführten Verbindungen I erhalten.

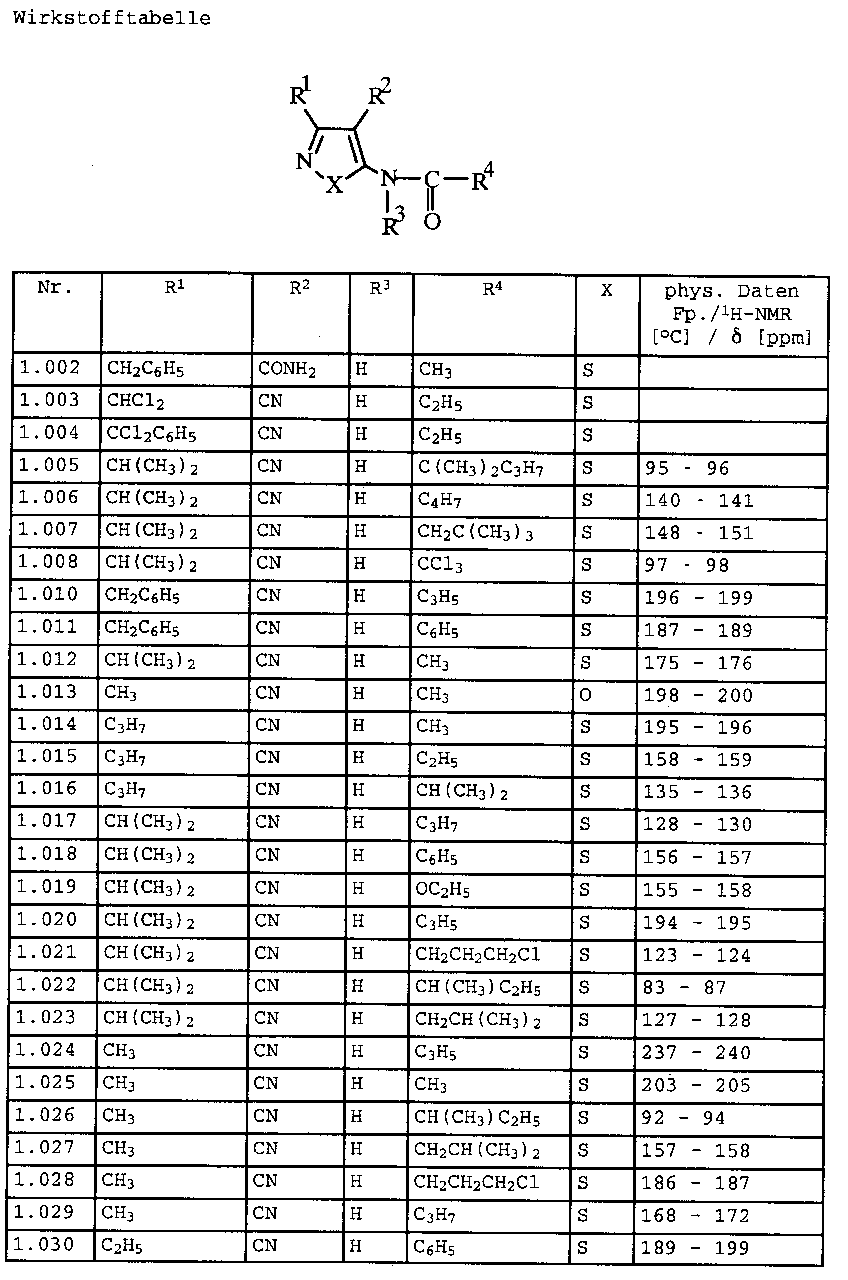

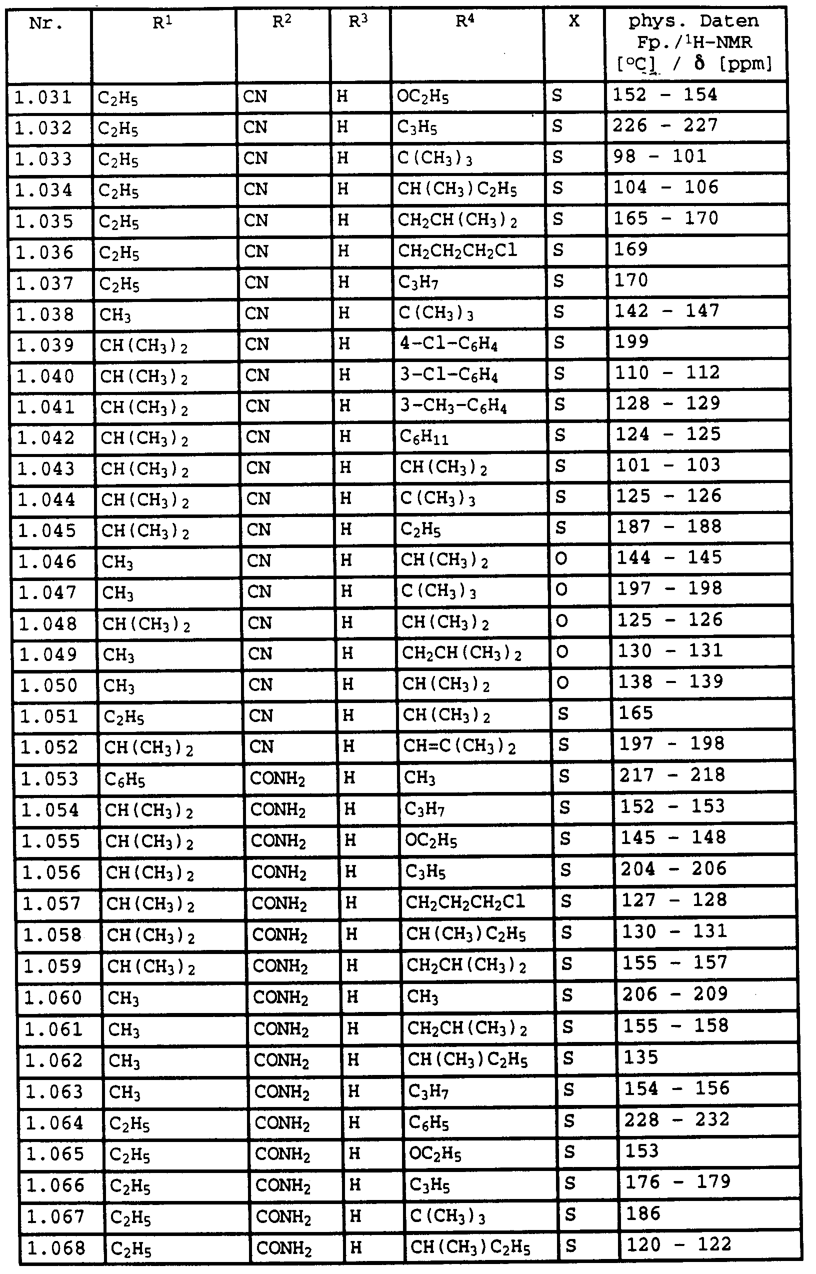

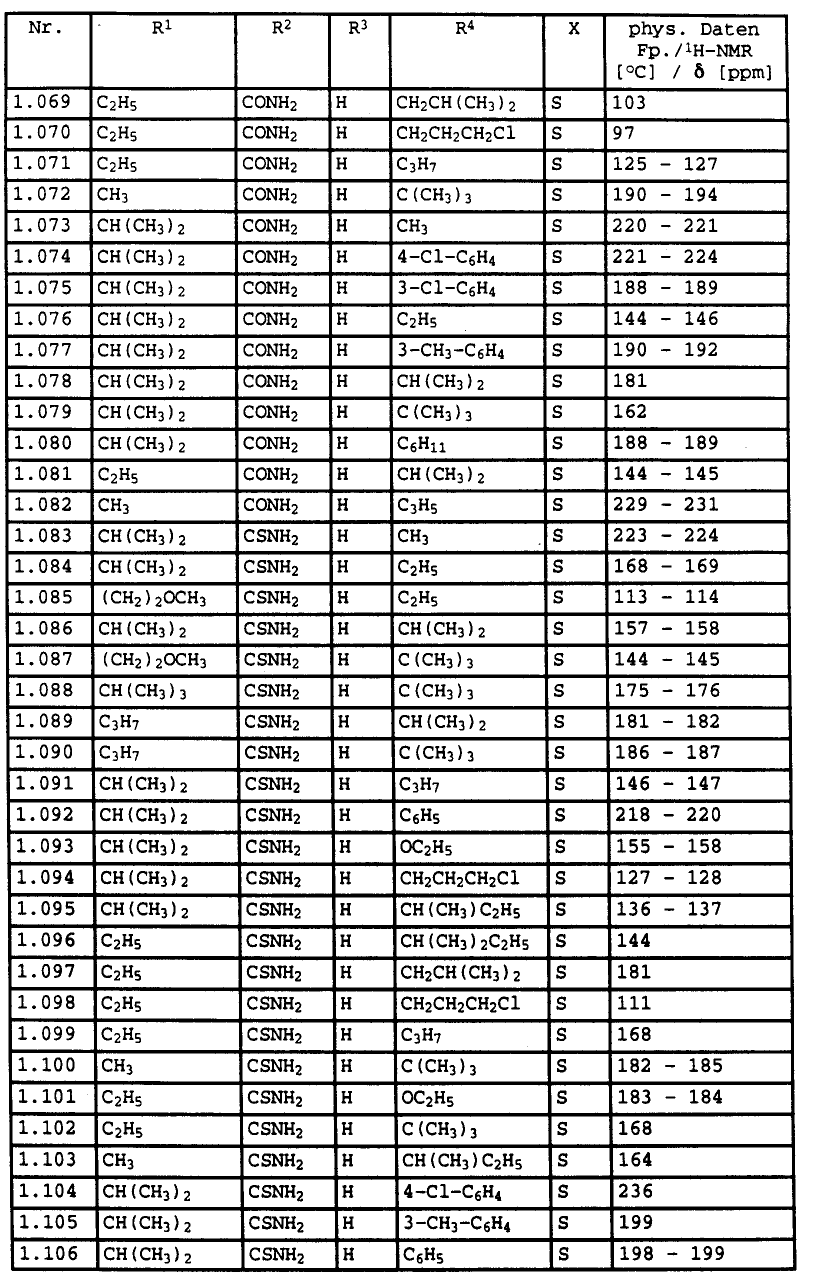

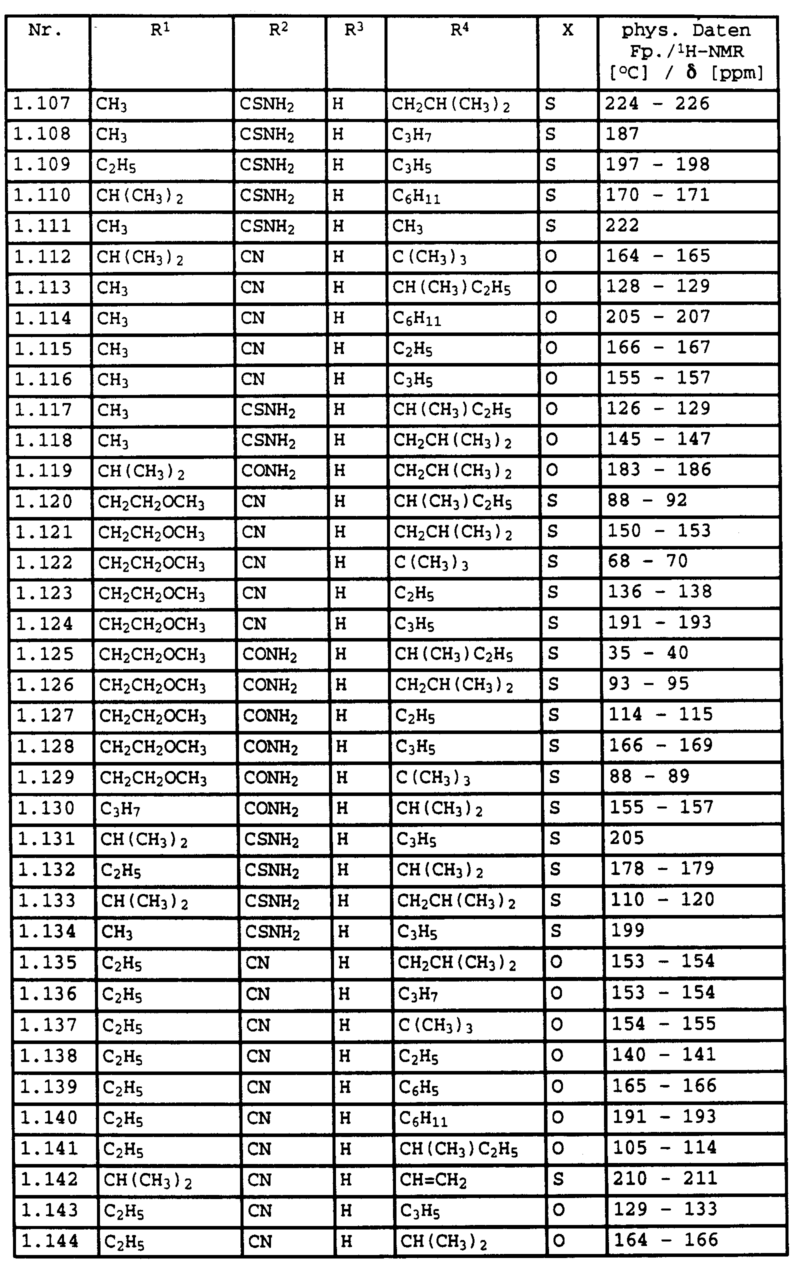

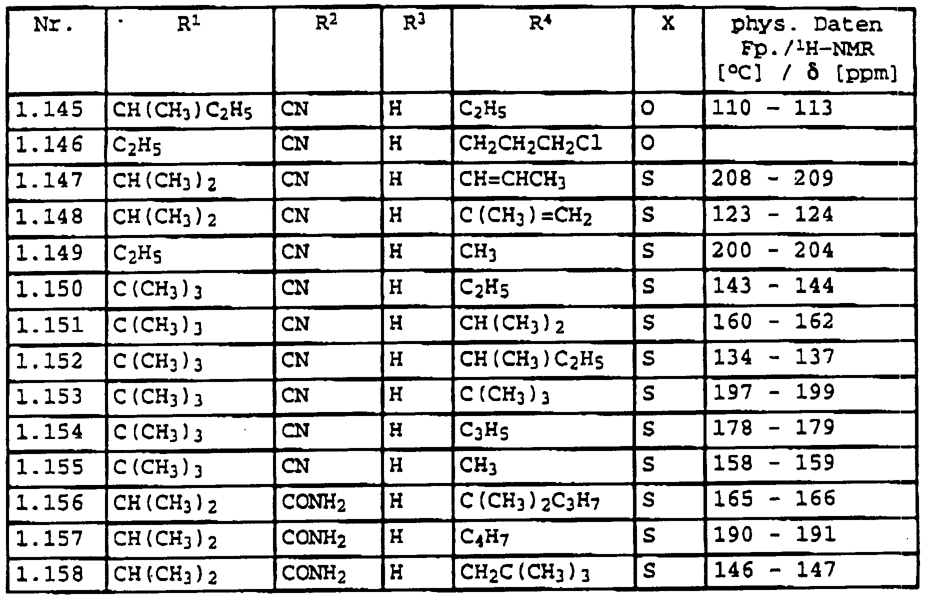

### Anwendungsbeispiele:

Die herbizide Wirkung der Acylamino-substituierten Isoxazol- bzw. Isothiazolderivate der Formel I auf das Wachstum der Testpflanzen ließ sich durch folgende Gewächshausversuche zeigen.

Als Kulturgefäße dienten Plastikblumentöpfe mit lehmigem Sand mit etwa 3,0 % Humus als Substrat. Die Samen dar Testpflanzen werden nach Arten getrennt eingesät.

Bei Vorauflaufßehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 4 bis 15 cm angezogen und erst dann mit den in Wasser suspendierten oder emulgierten Wirkstoffen behandelt. Die Testpflanzen öden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,25 kg/ha a.S..

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 keine Keimung der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Chenopodium album | Weißer Gänsefuß |
| Polygonum persicaria | Flohknöterich |

Mit 0,25 kg/ha a.S. im Nachauflaufverfahren eingesetzt ließen sich mit den Beispielen Nr. 1.043 und 1.044 unerwünschte Pflanzen sehr gut bekämpfen.

Im Vergleich zu strukturell ähnlichen Verbindungen des Standes der Technik, z.B. Verbindung A 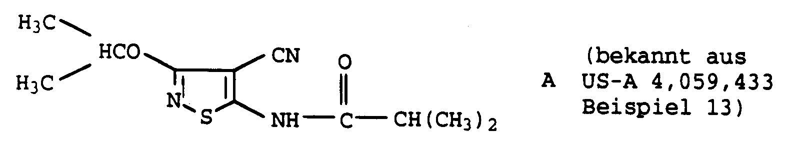 oder Verbindung B 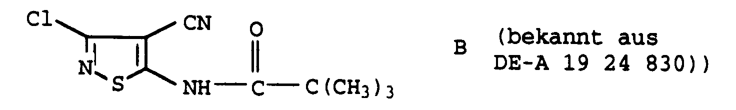 weisen die erfindungsgemäßen Verbindungen eine bessere herbizide Wirkung bzw. höhere Selektivität auf, wie die in nachfolgenden Tabellen I und II aufgeführten Resultate zeigen. Die Versuchsdurchführung erfolgte wie voranstehend beschrieben im Nachauflaufverfahren.

**Tabelle I**

| Herbizide Wirkung der Verbindungen 1.043 und Vergleichssubstanz A im Nachauflaufverfahren (Gewächshaus) | | | | |
|---|---|---|---|---|
| Verbindung | Aufwandmenge [kg/ha] a.i. | Testpflanzen [Schädigung in %] | | |
| | | ECHCG | ABUTH | IPOSS |
| 1.043 | 1,0 | 100 | 100 | 100 |
| | 0,5 | 100 | 100 | 100 |
| A | 1,0 | 20 | 15 | 40 |
| | 0,5 | 0 | 10 | 25 |

| | | | | |
|---|---|---|---|---|
| ECHCG = Echinochloa crus-galli | | | | |
| ABUTH = Abutilon theophrasti | | | | |
| IPOSS = Ipomoea ssp. | | | | |

**Tabelle II**

| Herbizide Wirkung und Selektivitäten der Verbindungen 1.044 und Vergleichssubstanz B im Nachauflaufverfahren (Gewächshaus) | | | | | | |
|---|---|---|---|---|---|---|
| Verbindung | Aufwandmenge [kg/ha] a.i. | Testpflanzen [Schädigung in %] | | | | |
| | | Kulturpflanzen | | unerwünschte Pflanzen | | |
| | | TRZAS | ZEAMX | ECHCG | SETVI | ABUTH |
| 1.044 | 1,0 | 0 | 10 | 100 | 100 | 100 |
| | 0,5 | 0 | 10 | 90 | 100 | 100 |
| B | 1,0 | 70 | 85 | 98 | 100 | 100 |
| | 0,5 | 50 | 85 | 98 | 100 | 100 |

| | | | | | | |
|---|---|---|---|---|---|---|
| TRZAS = Triticum aestivum (Sommerweizen) | | | | | | |
| ZEAMX = Zea mays (Mais) | | | | | | |
| ECHCG = Echinochloa crus-galli | | | | | | |
| SETVI = Setaria viridis | | | | | | |
| ABUTH = Abutilon theophrasti | | | | | | |

## Patentansprüche

1. Acylamino-substituierte Isoxazol- bzw. Isothiazolderivate der Formel I 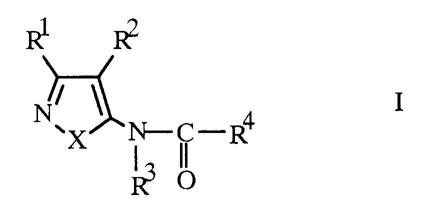 in der die Variablen folgende Bedeutung haben:
X
Sauerstoff oder Schwefel;
R¹
C₁-C₆-Alkyl, welches ein bis fünf Halogenatome und/oder einen Cyanorest und/oder bis zu zwei der folgenden Reste tragen kann: C₁-C₄-Alkoxy, C₁-C₄-Halogenalkoxy, C₁-C₄-Alkylthio, C₁-C₄-Halogenalkylthio, C₃-C₆-Cycloalkyl, welches wiederum durch ein bis drei Halogen- oder C₁-C₄-Alkylreste substituiert sein kann oder Phenyl, welches noch einen bis drei der folgenden Reste tragen kann: Cyano, Halogen, Nitro, C₁-C₆-Alkyl, C₁-C₆-Halogenalkyl, C₁-C₆-Alkoxy, C₁-C₆-Halogenalkyl, C₁-C₆-Alkylthio, C₁-C₆-Halogenalkylthio;
C₂-C₆-Alkenyl, dessen Doppelbindung epoxidiert sein kann, oder eine C₂-C₆-Alkinylgruppe, wobei beide Gruppen ein- bis dreimal durch Halogen, C₁-C₃-Alkoxy und/oder einmal durch Cyclopropyl oder Phenyl substituiert sein können, wobei der Phenylrest zusätzlich ein bis drei der folgenden Substituenten tragen kann: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄ -Halogenalkylthio;
C₃-C₈-Cycloalkyl oder C₃-C₆-Cycloalkenyl, wobei beide Gruppen ein-bis dreimal durch C₁-C₄-Alkyl und/oder Halogen substituiert sein können;
R²
CN, CONH₂, CSNH₂;
R³
Wasserstoff;
C₁-C₆-Alkyl, das einen bis drei der folgenden Substituenten tragen kann: Hydroxy, Halogen, C₁-C₄-Alkoxy und/oder C₁-C₄-Alkylthio;
C₃-C₈-Cycloalkyl, das ein- bis dreimal durch Halogen, C₁-C₄-Alkyl und/oder C₁-C₄-Halogenalkyl substituiert sein kann;
R⁴
eine C₁-C₄-Alkoxygruppe;
eine C₁-C₆-Alkylgruppe, die einen bis drei der folgenden Reste tragen kann: Halogen, Cyano, C₁-C₄-Alkoxy, C₁-C₄-Halogenalkoxy, C₁-C₄-Alkylthio, C₁-C₄-Halogenalkylthio, C₃-C₈-Cycloalkyl oder Phenyl, wobei der Phenylring seinerseits einen bis drei der folgenden Reste tragen kann: Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio und/oder C₁-C₄-Halogenalkylthio;
eine C₃-C₆-Cycloalkenylgruppe, die ein- bis dreimal durch Halogen oder C₁-C₄-Alkyl substituiert sein kann;
eine C₃-C₈-Cycloalkylgruppe, die einen bis drei der folgenden Reste tragen kann: Halogen, Nitro, Cyano, C₁-C₆-Alkyl, C₁-C₆-Halogenalkyl, C₁-C₄-Alkoxy und/oder C₁-C₄-Halogenalkoxy;
eine C₃-C₆-Alkenyl- oder C₃-C₆-Alkinylgruppe, die jeweils einbis dreimal durch Halogen und/oder einmal durch Phenyl substituiert sein können, wobei der Phenylrest seinerseits eine bis drei der folgenden Gruppen tragen kann: C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, Halogen, Cyano oder Nitro;
die Phenylgruppe, welche noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio und/oder C₁-C₆-Halogenalkylthio;
und landwirtschaftlich brauchbare Salze der Isoxazol- bzw. Isothiazolderivate I, ausgenommen:
3-Methyl-4-cyano-5-benzoylamino-isothiazol,
3-Methyl-4-carboxamido-5-acetylamino-isothiazol,
3-Methyl-4-carboxamido-5-(4-chlorbenzoyl)-amino-isothiazol,
3-Methyl-4-carboxamido-5-(2-chlorbenzoyl)-amino-isothiazol,
3-Methyl-4-carboxamido-5-benzoylamino-isothiazol.

2. Isoxazol- und Isothiazolderivate der Formel I gemäß Anspruch 1, in der die Reste R², R³ und R⁴ die in Anspruch 1 genannte Bedeutung haben und R¹ für eine C₂-C₆-Alkylgruppe steht oder eine Methylgruppe bedeutet, die substituiert ist durch ein bis drei Halogenatome und/oder einen Cyanorest und/oder bis zu zwei der folgenden Reste: C₁-C₄-Alkoxy, C₁-C₄-Halogenalkoxy, C₁-C₄-Alkylthio, C₁-C₄-Halogenalkylthio, C₃-C₆-Cycloalkyl, welches wiederum durch ein bis drei Halogen- und/oder Alkylreste substituiert sein kann oder Phenyl, welches noch einen bis drei der folgenden Reste tragen kann: Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio.

3. Isoxazol- und Isothiazolderivate der Formel I gemäß Anspruch 1,
in der die Variablen folgende Bedeutung haben:
X
Sauerstoff oder Schwefel;
R¹
C₂-C₆-Alkyl, C₂-C₆-Alkenyl, C₂-C₆-Alkinyl, C₃-C₈-Cycloalkyl oder C₃-C₆-Cycloalkenyl, wobei diese Gruppen jeweils wie in Anspruch 1 definiert substituiert sein können;
R²
CN, CONH₂ oder CSNH₂;
R³
Wasserstoff; C₁-C₆-Alkyl;
R⁴
C₁-C₄-Alkoxy, C₁-C₆-Alkyl oder C₃-C₈-Cycloalkyl, wobei die Alkyl- bzw. Cycloalkylreste wie in Anspruch 1 definiert substituiert sein können.

4. Verfahren zur Herstellung von Verbindungen der Formel I gemäß Anspruch 1,
in der X und R¹ bis R⁴ die oben genannten Bedeutungen haben, dadurch gekennzeichnet, daß man ein Amin der Formel II, 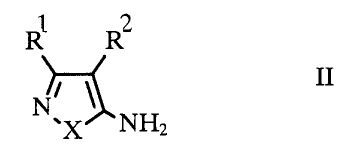 oder dessen Salz mit einem Carbonsäurechlorid der Formel III, 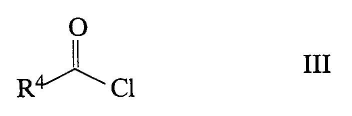 oder dem entsprechenden Anhydrid zu einem Amid der Formel IV umsetzt 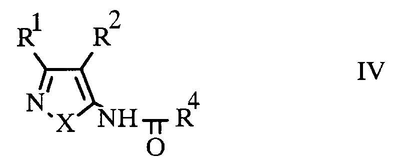 und dieses für den Fall R³ = substituiertes Alkyl oder Cycloalkyl anschließend in Gegenwart einer Base in an sich bekannter Weise mit Alkyl- bzw. Cycloalkylhalogeniden V
R³-Hal V
oder Alkyl- bzw. Cycloalkylsulfaten der Formel VI
(R³O)₂SO₂ VI
umsetzt.

5. Verfahren zur Herstellung von Verbindungen der Formel I gemäß Anspruch 1,
in der R² für CONH₂ steht und X, R¹, R³ und R⁴ die oben angegebenen Bedeutungen haben, dadurch gekennzeichnet, daß man eine Cyanoverbindung der allgemeinen Formel VII 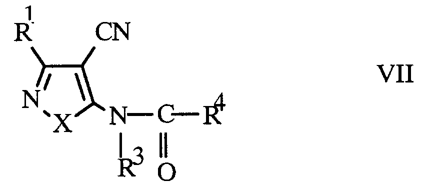 in an sich bekannter Weise hydrolysiert.

6. Verfahren zur Herstellung von Verbindungen der Formel I gemäß Anspruch 1
in der R² für CSNH₂ steht und X, R¹, R³ und R⁴ die oben angegebenen Bedeutungen haben, dadurch gekennzeichnet, daß man eine Cyanoverbindung der Formel VII 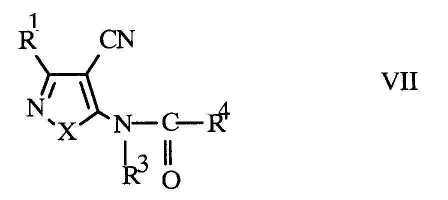 mit Schwefelwasserstoff behandelt.

7. Herbizide Mittel, enthaltend Acylamino-substituierte Isoxazol- bzw. Isothiazolderivate der Formel I gemäß den Ansprüchen 1 bis 3 sowie inerte Zusatzstoffe.

8. Verfahren zur Bekämpfung unerwünschten Pflanzenwuchses, dadurch gekennzeichnet, daß man die Pflanzen und/oder ihren Lebensraum mit einer herbizid wirksamen Menge eines Acylamino-substituierten Isoxazol- bzw. Isothiazolderivats der Formel I gemäß Anspruch 1 behandelt.

9. Verwendung der Acylamino-substituierten Isoxazol- bzw. Isothiazolderivate der Formel I gemäß den Ansprüchen 1 bis 3 als Herbizide.

## Claims

1. An acylamino-substituted isoxazole or isothiazole derivative of the formula I 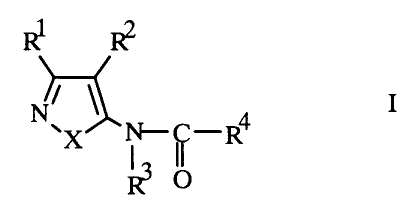 where
X
is oxygen or sulfur,
R¹
is C₁-C₆-alkyl which may carry from one to five halogen atoms or one cyano radical or up to two of the following radicals: C₁-C₄-alkoxy, C₁-C₄-haloalkoxy, C₁-C₄-alkylthio, C₁-C₄-haloalkylthio, C₃-C₆-cycloalkyl, which in turn may be substituted by from one to three halogen or C₁-C₄-alkyl radicals, or phenyl which may furthermore carry from one to three of the following radicals: cyano, halogen, nitro, C₁-C₆-alkyl, C₁-C₆-haloalkyl, C₁-C₆-alkoxy, C₁-C₆-haloalkoxy, C₁-C₆-alkylthio and C₁-C₆-haloalkylthio;
C₂-C₆-alkenyl, whose double bond may be epoxidized, or C₂-C₆-alkynyl, where both groups may be monosubstituted to trisubstituted by halogen or C₁-C₃-alkoxy or monosubstituted by cyclopropyl or phenyl, where the phenyl radical may additionally carry from one to three of the following substituents: halogen, cyano, nitro, C₁-C₄-alkyl, C₁-C₄-haloalkyl, C₁-C₄-alkoxy, C₁-C₄-haloalkoxy, C₁-C₄-alkylthio and C₁-C₄-haloalkylthio;
C₃-C₈-cycloalkyl or C₃-C₆-cycloalkenyl, where both groups may be monosubstituted to trisubstituted by C₁-C₄-alkyl or by halogen;
R²
is CN, CONH₂ or CSNH₂;
R³
is hydrogen;
C₁-C₆-alkyl which may carry from one to three of the following substituents: hydroxyl, halogen, C₁-C₄-alkoxy or C₁-C₄-alkylthio;
C₃-C₈-cycloalkyl which may be monosubstituted to trisubstituted by halogen, C₁-C₄-alkyl or C₁-C₄-haloalkyl;
R⁴
is C₁-C₄-alkoxy;
C₁-C₆-alkyl which may carry from one to three of the following radicals: halogen, cyano, C₁-C₄-alkoxy, C₁-C₄-haloalkoxy, C₁-C₄-alkylthio, C₁-C₄-haloalkylthio, C₃-C₈-cycloalkyl or phenyl, where the phenyl ring in turn may carry from one to three of the following radicals: halogen, cyano, nitro, C₁-C₄-alkyl, C₁-C₄-haloalkyl, C₁-C₄-alkoxy, C₁-C₄-haloalkoxy, C₁-C₄-alkylthio or C₁-C₄-haloalkylthio;
C₃-C₆-cycloalkenyl which may be monosubstituted to trisubstituted by halogen or by C₁-C₄-alkyl;
C₃-C₈-cycloalkyl which may carry from one to three of the following radicals: halogen, nitro, cyano, C₁-C₆-alkyl, C₁-C₆-haloalkyl, C₁-C₄-alkoxy or C₁-C₄-haloalkoxy;
C₃-C₆-alkenyl or C₃-C₆-alkynyl, each of which may be monosubstituted to trisubstituted by halogen or monosubstituted by phenyl, where the phenyl radical in turn may carry from one to three of the following groups: C₁-C₄-alkyl, C₁-C₄-haloalkyl, C₁-C₄-alkoxy, C₁-C₄-haloalkoxy, C₁-C₄-alkylthio, C₁-C₄-haloalkylthio, halogen, cyano or nitro;
phenyl which may furthermore carry from one to three of the following radicals: cyano, nitro, halogen, C₁-C₆-alkyl, C₁-C₆-haloalkyl, C₁-C₆-alkoxy, C₁-C₆-haloalkoxy, C₁-C₆-alkylthio or C₁-C₆-haloalkylthio;
and agriculturally useful salts of the isoxazole or isothiazole derivative I, with the exception of:
3-methyl-4-cyano-5-benzoylaminoisothiazole,
3-methyl-4-carboxamido-5-acetylaminoisothiazole,
3-methyl-4-carboxamido-5-(4-chlorobenzoyl)-aminoisothiazole,
3-methyl-4-carboxamido-5-(2-chlorobenzoyl)-aminoisothiazole
and 3-methyl-4-carboxamido-5-benzoylaminoisothiazole.

2. An isoxazole or isothiazole derivative of the formula I as claimed in claim 1, where R², R³ and R⁴ have the meaning stated in claim 1 and R¹ is C₂-C₆-alkyl or methyl which is substituted by from one to three halogen atoms or one cyano radical or up to two of the following radicals: C₁-C₄-alkoxy, C₁-C₄-haloalkoxy, C₁-C₄-alkylthio, C₁-C₄-haloalkylthio, C₃-C₆-cycloalkyl, which in turn may be substituted by from one to three halogen or alkyl radicals, or phenyl which may furthermore carry from one to three of the following radicals: cyano, nitro, halogen, C₁-C₆-alkyl, C₁-C₆-haloalkyl, C₁-C₆-alkoxy, C₁-C₆-haloalkoxy, C₁-C₆-alkylthio or C₁-C₆-haloalkylthio.

3. An isoxazole or isothiazole derivative of the formula I as claimed in claim 1,
where
X
is oxygen or sulfur;
R¹
is C₂-C₆-alkyl, C₂-C₆-alkenyl, C₂-C₆-alkynyl, C₃-C₈-cycloalkyl or C₃-C₆-cycloalkenyl, where each of these groups may be substituted as defined in claim 1;
R²
is CN, CONH₂ or CSNH₂;
R³
is hydrogen or C₁-C₆-alkyl and
R⁴
is C₁-C₄-alkoxy, C₁-C₆-alkyl or C₃-C₈-cycloalkyl, where the alkyl and cycloalkyl radicals may be substituted as defined in claim 1.

4. A process for the preparation of a compound of the formula I as claimed in claim 1,
where X and R¹ to R⁴ have the abovementioned meanings, wherein an amine of the formula II 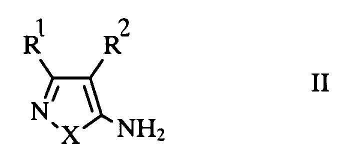 or a salt thereof is reacted with a carbonyl chloride of the formula III 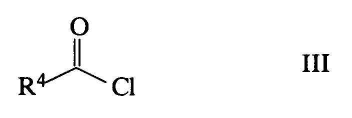 or with the corresponding anhydride to give an amide of the formula IV 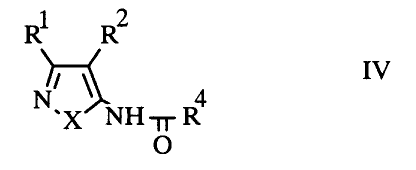 and, where R³ is substituted alkyl or cycloalkyl, this amide is then reacted in a manner known per se with an alkyl or cycloalkyl halide V
R³-Hal V
or an alkyl or cycloalkyl sulfate of the formula VI
(R³O)₂SO₂ VI
in the presence of a base.

5. A process for the preparation of a compound of the formula I as claimed in claim 1,
where R² is CONH₂ and X, R¹, R³ and R⁴ have the abovementioned meanings, wherein a cyano compound of the formula VII 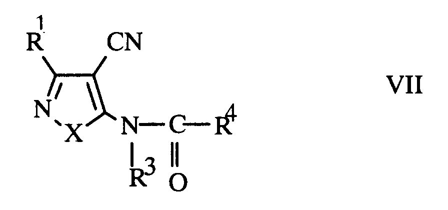 is hydrolyzed in a manner known per se.

6. A process for the preparation of a compound of the formula I as claimed in claim 1,
where R² is CSNH₂ and X, R¹, R³ and R⁴ have the abovementioned meanings, wherein a cyano compound of the formula VII 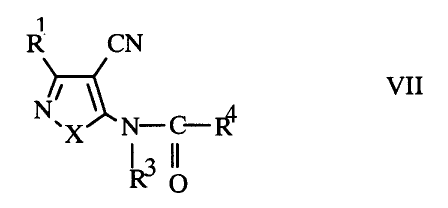 is treated with hydrogen sulfide.

7. A herbicide containing an acylamino-substituted isoxazole or isothiazole derivative of the formula I as claimed any of claims 1 to 3 and inert additives.

8. A method for controlling undesirable plant growth, wherein the plants or their habitat are treated with a herbicidally effective amount of an acylamino-substituted isoxazole or isothiazole derivative of the formula I as claimed in claim 1.

9. Use of an acylamino-substituted isoxazole or isothiazole derivative of the formula I as claimed in any of claims 1 to 3 as a herbicide.

## Revendications

1. Dérivés de l'isoxazole et de l'isothiazole à substituants acylamino, répondant à la formule I 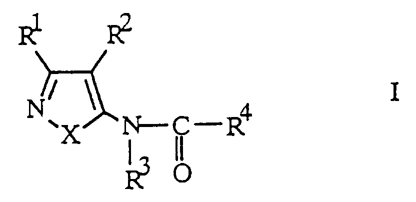 dans laquelle les symboles ont les significations suivantes :
X
l'oxygène ou le soufre ;
R¹
un groupe alkyle en C1-C6 qui peut porter un à cinq atomes d'halogènes et/ou un groupe cyano et/ou jusqu'à deux des substituants suivants : alcoxy en C1-C4, halogénoalcoxy en C1-C4, alkylthio en C1-C4, halogénoalkylthio en C1-C4, cycloalkyle en C3-C6, lequel peut lui-même porter un à trois substituants halogéno ou alkyle en C1-C4, ou phényle, lequel peut lui-même porter un à trois des substituants suivants : cyano, halogéno, nitro, alkyle en C1-C6, halogénoalkyle en C1-C6, alcoxy en C1-C6, halogénoalkyle en C1-C6, alkylthio en C1-C6, halogénoalkylthio en C1-C6 ;
un groupe alcényle en C2-C6, dont la double liaison peut être époxydée, ou un groupe alcynyle en C2-C6, chacun de ces groupes pouvant porter un à trois substituants halogéno, alcoxy en C1-C3 et/ou un substituant cyclopropyle ou phényle, ce dernier pouvant en outre porter un à trois des substituants suivants : halogéno, cyano, nitro, alkyle en C1-C4, halogénoalkyle en C1-C4, alcoxy en C1-C4, halogénoalcoxy en C1-C4, alkylthio en C1-C4, halogénoalkylthio en C1-C4 ;
un groupe cycloalkyle en C3-C8 ou cycloalcényle en C3-C6, chacun de ces groupes pouvant porter un à trois substituants alkyle en C1-C4 et/ou halogéno ;
R²
CN, CONH₂, CSNH₂ ;
R³
l'hydrogène ;
un groupe alkyle en C1-C6 qui peut porter un à trois des substituants suivants : hydroxy, halogéno, alcoxy en C1-C4 et/ou alkylthio en C1-C4 ;
un groupe cycloalkyle en C3-C8 qui peut porter un à trois substituants halogéno, alkyle en C1-C4 et/ou halogénoalkyle en C1-C4 ;
R⁴
un groupe alcoxy en C1-C4 ;
un groupe alkyle en C1-C6 qui peut porter un à trois des substituants suivants : halogéno, cyano, alcoxy en C1-C4, halogénoalcoxy en C1-C4, alkylthio en C1-C4, halogénoalkylthio en C1-C4, cycloalkyle en C3-C8 ou phényle, le cycle phényle pouvant lui-même porter un à trois des substituants suivants : halogéno, cyano, nitro, alkyle en C1-C4, halogénoalkyle en C1-C4, alcoxy en C1-C4, halogénoalcoxy en C1-C4, alkylthio en C1-C4 et/ou halogénoalkylthio en C1-C4 ;
un groupe cycloalcényle en C3-C6 qui peut porter un à trois des substituants halogéno ou alkyle en C1-C4 ;
un groupe cycloalkyle en C3-C8 qui peut porter un à trois des substituants suivants : halogéno, nitro, cyano, alkyle en C1-C6, halogénoalkyle en C1-C6, alcoxy en C1-C4 et/ou halogénoalcoxy en C1-C4 ;
un groupe alcényle en C3-C6 ou alcynyle en C3-C6, chacun d'eux pouvant porter un à trois substituants halogéno et/ou un substituant phényle, le groupe phényle pouvant lui-même porter un à trois des substituants suivants : alkyle en C1-C4, halogénoalkyle en C1-C4, alcoxy en C1-C4, halogénoalcoxy en C1-C4, alkylthio en C1-C4, halogénoalkylthio en C1-C4, halogéno, cyano ou nitro ;
le groupe phényle qui peut lui-même porter un à trois des substituants suivants : cyano, nitro, halogéno, alkyle en C1-C6, halogénoalkyle en C1-C6, alcoxy en C1-C6, halogénoalcoxy en C1-C6, alkylthio en C1-C6 et/ou halogénoalkylthio en C1-C6 ; et les sels des dérivés de l'isoxazole et de l'isothiazole I aptes aux applications agricoles, à l'exception des suivants :
3-méthyl-4-cyano-5-benzoylamino-isothiazole,
3-methyl-4-carboxamido-5-acétylamino-isothiazole,
3-méthyl-4-carboxamido-5-(4-chlorobenzoyl)amino-isothiazole,
3-méthyl-4-carboxamido-5-(2-chlorobenzoyl)-amino-isothiazole,
3-méthyl-4-carboxamido-5-benzoylamino-isothiazole,

2. Dérivés de l'isoxazole et de l'isothiazole de formule I de la revendication 1, dans laquelle les symboles R², R³ et R⁴ ont les significations indiquées dans la revendication 1, et R¹ représente un groupe alkyle en C2-C6 ou un groupe méthyle qui porte un à trois substituants halogéno et/ou un substituant cyano et/ou jusqu'à deux des substituants suivants : alcoxy en C1-C4, halogénoalcoxy en C1-C4, alkylthio en C1-C4, halogénoalkylthio en C1-C4, cycloalkyle en C3-C6 lequel peut lui-même porter un à trois des substituants halogéno et/ou alkyle, ou phényle, lequel peut encore porter un à trois des substituants suivants : cyano, nitro, halogéno, alkyle en C1-C6, halogénoalkyle en C1-C6, alcoxy en C1-C6, halogénoalcoxy en C1-C6, alkylthio en C1-C6, halogénoalkylthio en C1-C6.

3. Dérivés de l'isoxazole et de l'isothiazole de formule I selon la revendication 1 dans laquelle les symboles ont les significations suivantes :
X
l'oxygène ou le soufre ;
R¹
un groupe alkyle en C2-C6, alcényle en C2-C6, alcynyle en C2-C6, cycloalkyle en C3-C8 ou cycloalcényle en C3-C6, chacun de ces groupes pouvant être substitué comme indiqué dans la revendication 1 ;
R²
CN, CONH₂ ou CSNH₂ ;
R³
l'hydrogène ; un groupe alkyle en C1-C6 ;
R⁴
un groupe alcoxy en C1-C4, alkyle en C1-C6 ou cycloalkyle en C3-C8, les groupes alkyle et cycloalkyle pouvant être substitués comme indiqué dans la revendication 1.

4. Procédé de préparation des composés de formule I de la revendication 1 dans laquelle X et R¹ à R⁴ ont les significations indiquées ci-dessus, caractérisé par le fait que l'on fait réagir une amine de formule II 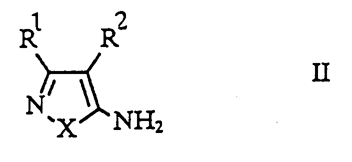 ou son sel, avec un chlorure d'acide carboxylique de formule III 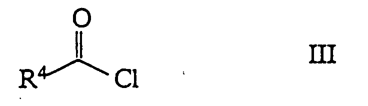 ou l'anhydride correspondant, ce qui donne un amide de formule IV 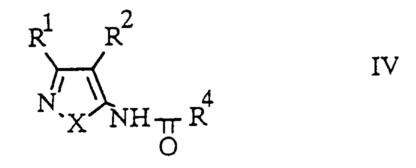 lequel, dans le cas où R³ = alkyle ou cycloalkyle substitué, est ensuite mis à réagir en présence d'une base, de manière connue en soi, avec des halogénures d'alkyle ou de cycloalkyle respectivement V
R³-Hal V
ou des sulfates d'alkyle ou de cycloalkyle respectivement de formule VI
(R³O)₂SO₂ VI

5. Procédé de préparation des composés de formule I de la revendication 1 dans laquelle R² représente CONH₂ et X, R¹ R³ et R⁴ ont les significations indiquées ci-dessus, caractérisé par le fait que l'on hydrolyse de manière connue en soi un dérivé cyané de formule générale VII

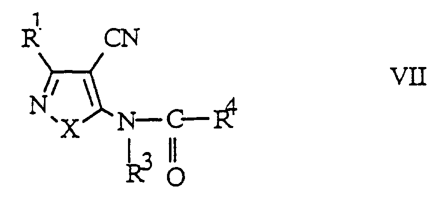

6. Procédé de préparation des composés de formule I de la revendication 1 dans laquelle R² représente CSNH₂ et X, R¹ R³ et R⁴ ont les significations indiquées ci-dessus, caractérisé par le fait que l'on traite un dérivé cyané de formule VII 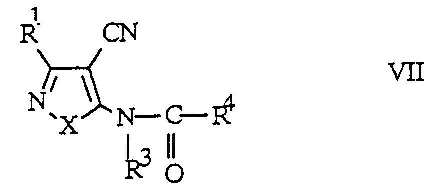 par le sulfure d'hydrogène.

7. Produits herbicides contenant des dérivés de l'isothiazole ou de l'isothiazole à substituants acylamino de formule I selon les revendications 1 à 3 et des additifs inertes.

8. Procédé pour combattre les croissances de végétaux indésirables, caractérisé par le fait que l'on traite les végétaux et/ou leur habitat par une quantité herbicide efficace d'un dérivé de l'isoxazole ou de l'isothiazole à substituant acylamino de formule I selon la revendication 1.

9. Utilisation des dérivés de l'isoxazole et de l'isothiazole à substituants acylamino de formule I selon les revendications 1 à 3 en tant qu'herbicides.
